(19) Europäisches Patentamt
European Patent Office
Office européen des brevets

(11) Numéro de publication: **0 195 009**
**B1**

(12) # FASCICULE DE BREVET EUROPEEN

(45) Date de publication du fascicule du brevet: **06.09.89**

(51) Int. Cl.⁴: **C 07 H 21/00**, G 01 N 33/48, C 12 Q 1/00

(21) Numéro de dépôt: **85902581.9**

(22) Date de dépôt: **14.06.85**

(86) Numéro de dépôt international: **PCT/FR 85/00151**

(87) Numéro de publication internationale: **WO 86/00074 (03.01.86 Gazette 86/1)**

(54) **ACIDES NUCLEIQUES MARQUES CHIMIQUEMENT, LEUR UTILISATION ET NECESSAIRE POUR SA MISE EN OEUVRE.**

(30) Priorité: **15.06.84 FR 8409421**

(43) Date de publication de la demande: **24.09.86 Bulletin 86/39**

(45) Mention de la délivrance du brevet: **06.09.89 Bulletin 89/36**

(84) Etats contractants désignés: **AT BE CH DE FR GB IT LI LU NL SE**

(56) Documents cités:
**EP-A- 0 097 373**

**Chemical Abstracts, vol. 99, no. 21, 21 NOvember 1983 (Columbus, Ohio, US), see page 331, no. 172313q. M. TALPAERT-BORLE et al.: "Reaction of apurinic/apyrimidinic sites with (14C9 methoxyamine. A method for the quantitative assay of SP sites in DNA"**

(73) Titulaire: **INSTITUT NATIONAL DE LA SANTE ET DE LA RECHERCHE MEDICALE (INSERM), 101, rue de Tolbiac, F-75654 Paris Cédex 13 (FR)**

(72) Inventeur: **TCHEN, Paul, 18, rue du Télégraphe, F-92000 Nanterre (FR)**

(74) Mandataire: **Ayache, Monique et al, CABINET AYACHE 3, côte de la Jonchère B.P. 24, F-92502 Rueil-Malmaison Cedex (FR)**

**EP 0 195 009 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un acide nucléique modifié, ou une séquence d'un acide, sa préparation, son utilisation pour l'étude, notamment la détection, la localisation, la caractérisation et/ou la purification d'un acide nucléique ou fragment d'acide nucléique, notamment d'un gène déterminé. Elle concerne encore l'utilisation de tels acides nucléiques modifiés pour la séparation ou la purification d'une molécule d'origine biologique présentant une affinité pour l'acide nucléique ou le fragment modifié. Elle s'étend également à des nécessaires ou «kits» permettant la mise en œuvre de telles utilisations.

Tout organisme vivant possède un patrimoine génétique constitué d'une ou plusieurs molécules d'acides nucléiques. L'enchaînement des constituants élémentaires de ces molécules (les désoxyribonucléotides ou les ribonucléotides) permet la formation de séquences d'une grande diversité. Ces séquences portent de façon codée les informations nécessaires à la vie; elles sont caractéristiques de l'organisme. L'étude, notamment la détection, la localisation, la caractérisation et/ou la purification des acides nucléiques et de leurs fragments, notamment des gènes, présentent un intérêt croissant dans la recherche de certaines anomalies congénitales, notamment chez l'être humain ou l'animal ou le diagnostic rapide d'une maladie, notamment infectieuse, par identification in vitro dans un échantillon biologique de séquences caractéristiques, présentes parmi un grand nombre d'autres séquences.

Pour la détection, voire l'enrichissement d'une composition d'acides nucléiques en un acide nucléique déterminé, il est connu d'avoir recours à des techniques d'hybridation entre l'acide nucléique déterminé recherché et une «sonde» portant un marqueur permettant ultérieurement de la repérer.

De telles sondes ont déjà été décrites: elles sont en général constituées par des acides ribonucléiques (ARN) ou désoxyribonucléiques (ADN). On sait que les ARN ont des séquences de nucléotides complémentaires de celles des ADN dont ils dérivent, cette complémentarité se manifestant par la capacité qu'ont ces ARN de former des hybrides mixtes avec les séquences correspondantes de ces ADN préalablement dénaturés, pour autant que ceux-ci étaient initialement bicaténaires, par exemple après incubation dans un milieu de haute force ionique et à température élevée. Ces sondes peuvent encore être constituées par des ADN de séquences déterminées qui, le cas échéant après une dénaturation préalable, peuvent s'hybrider avec des acides nucléiques ayant des séquences complémentaires.

Les acides nucléiques peuvent aussi être utilisés pour localiser ou pour purifier des gènes. Fixés à des supports solides, ils peuvent aussi servir à la purification de molécules présentant une affinité pour les acides nucléiques telles que les nucléases, polymérases, gyrases et autres enzymes utilisant les acides nucléiques comme substrat. D'autres applications peuvent être envisagées, par exemple des filtres retenant spécifiquement les ARN messagers peuvent être fabriqués en liant des poly-uridylates ou des poly-thymidylates à des filtres de nylon ou de cellulose.

Mais les acides nucléiques sont extrêmement inertes sur le plan chimique. On connaît les difficultés qu'implique leur couplage avec une autre molécule, par exemple un marqueur – en particulier dans le cas de marqueurs non radio-actifs – ou avec toute autre forme de support.

On connaît, à l'heure actuelle, peu de techniques permettant la réalisation de couplages efficaces. Dans le brevet français 7 810 975 (publié sous le numéro 2 422 956) il a été proposé de coupler des ADN avec des molécules telles que la biotine ou l'avidine par l'intermédiaire d'un complexe mettant en jeu des groupes dérivés de Cytochrome C. La liaison obtenue peut ne pas toujours présenter la solidité voulue, dans le cas où les acides nucléiques en cause doivent être mis en œuvre dans des techniques de purification du genre de celles qui ont été évoquées plus haut. D'autres procédés comprennent la modification préalable de nucléotides isolés, puis leur insertion par des réactions enzymatiques dans la chaîne de l'acide nucléique que l'on souhaite modifier, notamment par remplacement de certains des nucléotides constitutifs de la chaîne par les nucléotides préalablement modifiés. Une telle technique a été décrite dans la demande de brevet européen n° 63 879. Cette technique, appliquée à des ADN bi-caténairisés, consiste à les mettre en contact dans un milieu approprié avec des mono-nucléotides préalablement modifiés en présence d'une nucléase et d'une polymérase. La fonction de la nucléase est alors «d'extraire» certains des nucléotides constitutifs de l'un ou de l'autre des brins de l'acide nucléique bi-caténaire et celle de la polymérase de promouvoir l'insertion d'autres nucléotides, parmi lesquels les nucléotides préalablement modifiés. Cette technique, pour efficace qu'elle soit, reste extrêmement délicate à mettre en œuvre.

L'invention a pour but de remédier à la fois à l'inertie chimique des acides nucléiques et aux difficultés rencontrées jusqu'à ce jour pour réaliser des modifications de leur structure, permettant leur couplage ultérieur à d'autres molécules ou à des supports. En particulier, l'invention a pour but de fournir un procédé particulièrement simple d'introduction de groupes de couplage dans des acides nucléiques tout en préservant leur capacité d'hybridation avec des séquences nucléiques complémentaires, elles-mêmes modifiées ou non.

L'invention met à profit la découverte qui a été faite que la capacité d'hybridation d'une séquence nucléotidique déterminée avec une séquence complémentaire n'impliquait pas la nécessaire conservation du caractère intact de tous ses groupes cytidine, thymidine, et/ou uridine. En particulier, la capacité d'hybridation des acides nucléiques n'est pas substantiellement affectée par la dégradation partielle de ses nucléosides

pyrimidiques, telles que thymidine et/ou désoxycytidine, en ce qui concerne les ADN, ou les groupes uridine ou cytidine en ce qui concerne les ARN. De tels acides nucléiques partiellement dégradés, mais conservant leur caractère polymérique, sont quelquefois appelés «acides apyrimidiques».

On savait déjà que de tels acides apyrimidiques pouvaient être obtenus par le traitement d'un acide désoxyribonucléique (ADN) par l'hydrazine (voir notamment A.R. Cashmore et Coll., Biochim. Biophys. Acta, 174, (1969), p. 591-603). Ces «acides apyrimidiques» comprennent, à la place d'au moins certains groupements désoxycytidine ou thymidine, des groupes:

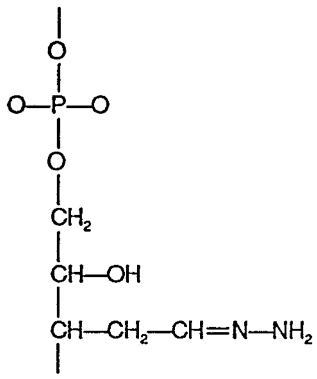

(I)

qui, dans leurs chaînes remplacent au moins certains des groupements désoxycytidine ou thymidine, conformément à ce qui a été établi par A. Temperli et Coll., Biochim. Biophys. Acta, 91, (1964), p. 462-476.

Mais les études effectuées par A.R. Cashmore et Coll. et A. Temperli et Coll. avaient pour but essentiel l'étude de la structure des nucléotides puriques dans les acides nucléiques concernés.

Par ailleurs, l'extrait des Chemical Abstracts vol. 99, (1983), n° 172 313q qui résume l'article Biochem. Biophys. Acta (1983), 740(4), 410-16 décrit une méthode de dosage des sites apuriniques/apyrimidiques sur des acides nucléiques dégradés en utilisant la réaction des groupements aldéhydes ainsi libérés avec la ($^{14}C$) méthoxyamine.

Une autre application importante de la réaction d'attaque des acides nucléiques par l'hydrazine, impliquant par conséquent le passage par un acide apyrimidique, s'adresse à l'étude des séquences des ADN.

Selon la méthode bien connue de A.M. MAXAM et W. GILBERT, Proc. Natl. Acad. Sci. USA, 74 (2), (1977), p. 560-564, l'hydrazine est utilisée pour cliver les bases thymine et cytosine, l'action de l'hydrazine étant ensuite relayée par celle de la pipéridine qui coupe l'acide nucléique au niveau des bases qui avaient été touchées, sépare tous les produits de réaction à l'hydrazine des sucres et catalyse la β-élimination des phosphates.

Il n'avait cependant jamais été proposé, ni même suggéré, de substituer des composés de type «acide apyrimidiques» à des acides nucléiques correspondants dans des applications du type de celles qui ont été évoquées plus haut.

Selon l'un de ses aspects, l'invention a pour objet l'application des acides apyrimidiques à la constitution de sondes servant à la détection et le cas échéant, à la séparation des séquences nucléotidiques d'acides nucléiques complémentaires contenus dans un milieu, par hybridation desdits acides apyrimidiques avec lesdites séquences nucléotidiques.

Cette substitution est particulièrement avantageuse en ce que les acides apyrimidiques, sont porteurs de groupes particulièrement réactifs, notamment de groupes $NH_2$ qui peuvent être mis à profit pour la fixation sur ces acides apyrimidiques de tout groupe de couplage approprié. Ces acides apyrimidiques ainsi modifiés constituent par conséquent des produits nouveaux aisément accessibles et conservant leur capacité d'hybridation avec les acides nucléiques complémentaires de ceux dont ils sont dérivés. Pour la commodité du langage, il va de soi que lorsque dans la suite on fera référence à «l'acide nucléique complémentaire» de l'acide apyrimidique, modifié par un ou plusieurs groupes de couplage conformes à l'invention, il faudra naturellement entendre qu'il s'agit de l'acide nucléique complémentaire de celui dont est dérivé l'acide apyrimidique modifié du genre en question.

La structure des acides nucléiques modifiés (ou acides apyrimidiques modifiés) par des groupes de couplage, conformes à l'invention, est donc dérivée d'une chaîne d'acide désoxyribonucléique (ou ribonucléique) et caractérisée par le remplacement d'une partie au moins des groupes désoxycytidine-phosphate (ou cytidine-phosphate) et, le cas échéant, d'au moins une partie des groupes thymidine-phosphate (ou uridine-phosphate) par des groupes:

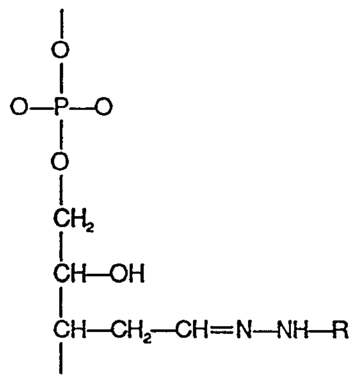

(II)

reliés dans la chaîne à des groupes semblables ou à des nucléotides intacts, R représentant un groupe de couplage.

Il est à remarquer que la totalité des groupes thymidine-phosphate (ou uridine-phosphate) peut être intacte, notamment lorsque le traitement de l'acide nucléique de départ à l'hydrazine a été réalisé en présence d'une concentration du milieu en chlorure de sodium suffisante pour protéger les groupes thymine (ou uracile) de l'action de l'hydrazine. On rappelle que dans la méthode

de MAXAM et GILBERT la présence de NaCl à une concentration 2M permet l'ouverture sélective des groupes cytosine.

Dans ce qui précède on entend par «groupe de couplage» tout substituant ou groupe dont la présence n'interfère pas avec la capacité de l'acide apyrimidique de s'hybrider avec un acide nucleique complementaire. Des exemples de tels groupes ont été décrits par exemple dans la demande de brevet européen n° 63 879 déjà mentionnée plus haut. Lorsque l'acide apyrimidique modifié selon l'invention est destiné à être utilisé en tant que sonde, le groupe de couplage est choisi parmi ceux qui peuvent être aisément couplés, directement ou indirectement à un marqueur, tels qu'une enzyme, une molécule fluorescente ou tout autre marqueur aisément visualisable.

A titre d'exemple, on indiquera que le groupe de couplage peut être avantageusement constitué par de la biotine, dont la présence peut être mis en évidence en mettant à profit la réaction spécifique biotine-avidine. Des modes de réalisation particuliers d'utilisation d'un acide nucléique modifié selon l'invention, portant des substituants biotine seront explicités plus loin en relation avec les applications d'un tel acide nucléique. A l'inverse, le groupe de couplage peut être constitué par l'avidine, la biotine étant alors mise en œuvre pour la détection de sondes hybridées porteuses de groupes avidine.

D'autres groupes de couplage formant des complexes caracteristiques tels que la ferritine peuvent être utilisés.

Le groupe de couplage peut être constitué par un antigène ou un haptène dont la présence peut être mise en évidence au moyen d'anticorps spécifiques de cet antigène ou de cet haptène selon des méthodes connues. A titre de haptènes et d'antigènes convenant à cet effet, on citera de façon non limitative la biotine, le groupement dinitrophényle, différents peptides, glycopeptides ou polypeptides. Avantageusement le groupe de couplage peut être constitué par une substance du type «anticorps universel» telle que la protéine A.

Selon un autre mode de réalisation, le groupe de couplage permet la liaison avec un support solide, constitué entre autres par la résine synthétique de type polyamide connue sous la dénomination de NYLON, de la cellulose, de l'agarose ou du latex.

D'une façon générale, des produits préférés de l'invention peuvent encore être représentés par la formule générale:

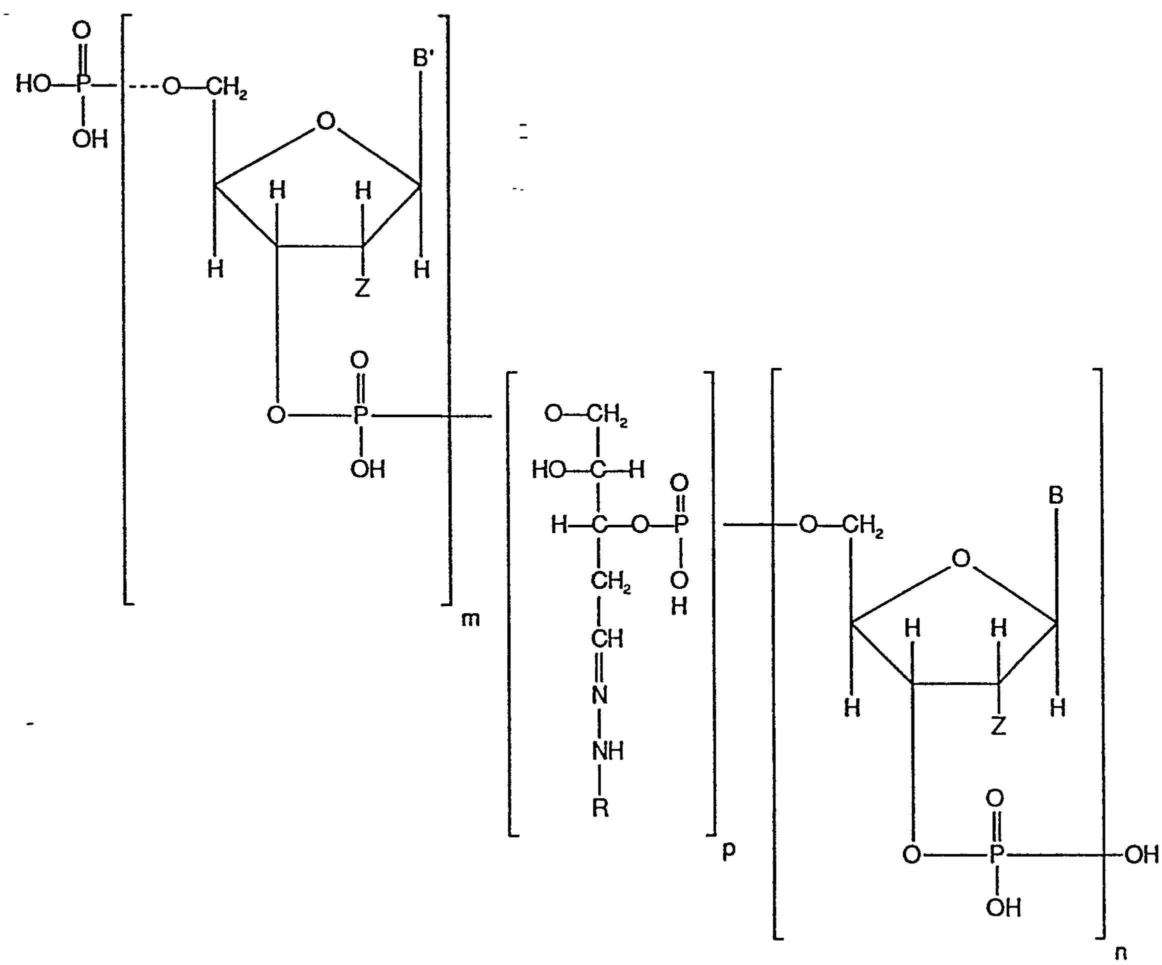

(III)

dans laquelle:

- chacune des lettres B, B' ... représente un groupe purine, 7-déazapurine ou pyrimidine, chacun de ces groupes B, B' ... étant lié de façon covalente au groupe sucre correspondant en la position $C^1$ de celui-ci, soit au niveau de sa propre position $N^9$, pour les groupes purine ou 7-déazapurine, soit au niveau de sa propre position $N^1$ pour les groupes pyrimidine,
- R représente un groupe de couplage reconnaissable par une molécule affine, un polypeptide ou un anticorps, ou encore un groupe permettant le couplage covalent avec un autre groupe fonctionnel porté soit par une autre molécule, soit par un support,
- z représente -H ou -OH et
- m et n représentent chacun O ou de nombres entiers et la somme m+n est avantageusement comprise entre 100 et 10000, de préférence comprise entre 300 et 2000,
- p est un nombre entier au moins égal à 1 et le rapport

$$\frac{p}{m+n+p}$$

est avantageusement compris entre 0,01 et 0,05.

L'acide apyrimidique modifié selon l'invention peut être obtenu par traitement par l'hydrazine d'un acide nucléique ou d'un fragment d'acide nucléique, avec fixation ultérieure du groupe de couplage, notamment en ayant recours aux méthodes connues de conjugaison des protéines.

Avantageusement, l'acide nucléique est fragmenté en fragments contenant par exemple de 300 à 2000 paires de bases. La fragmentation peut être effectuée par sonication.

La quantité d'hydrazine à utiliser dépend essentiellement de la quantité de sites réactifs (groupements $= N-NH_2$) que l'on veut obtenir. A titre indicatif, on obtient des résultats intéressants en utilisant de 0,5 à 4 µl d'hydrazine anhydre par µg d'acide nucléique en solution aqueuse à une concentration de 0,5 mg/ml.

Après élimination de l'hydrazine en excès, par exemple par précipitation de l'acide nucléique modifié par l'hydrazine à l'éthanol froid, ou extraction de l'hydrazine au moyen d'un solvant non miscible à l'eau tel que par exemple l'éther éthylique, on effectue la fixation du groupe de couplage désiré sur les fonctions amine libre introduites dans l'acide nucléique par le traitement à l'hydrazine.

Cette fixation peut être effectuée selon des méthodes connues de conjugaison des protéines. A titre indicatif:

- on peut condenser l'acide apyrimidique avec un composé portant au moins une fonction amine libre, en présence de glutaraldéhyde;
- on peut condenser l'acide apyrimidique avec un composé portant au moins une fonction carboxyle libre en présence d'un carbodiimide hydrosoluble, en phase aqueuse, ou d'un carbodiimide liposoluble, au sein d'un solvant organique inerte tel que par exemple le diméthyl-formamide (DMF), le tétrahydrofuranne (THF) ou le toluène; des carbodiimides hydrosolubles avantageux sont le N-cyclohexyl-N'-[(3-(N-méthyl-morpholino)éthyl] carbodiimide p-toluène sulfonate et le chlorhydrate de (3-méthyl)-aminopropyl-carbodiimide; le dicyclohexyl-carbodiimide, entre autres, est approprié pour des réactions au sein d'un solvant organique;
- on peut également condenser l'acide apyrimidique avec un composé portant au moins une fonction carboxyle, en faisant d'abord réagir ce dernier avec un chlorocarbonate d'alcoyle (en $C_2$ à $C_4$), par exemple dans le toluène en présence d'une amine tertiaire, puis en faisant réagir l'anhydride mixte ainsi obtenu avec l'acide nucléique modifié à l'hydrazine;
- on peut encore condenser l'acide apyrimidique avec un composé portant au moins une fonction carboxyle en faisant réagir un ester activé de ce dernier, par exemple son ester succinimidique ou N-hydroxysuccinimidique, ou son produit de réaction avec le p-nitrophénol, dans un solvant organique inerte présentant de préférence un caractère polaire; le diméthyl-formamide est préféré;
- on peut condenser l'acide apyrimidique avec un composé portant au moins une fonction aldéhyde, par formation d'une base de Schiff entre les deux réactifs avec réduction éventuelle, par exemple selon la méthode décrite par G.R. Gray dans Arch. Biochim. Biophys. 163 (1974), p. 425.

L'invention a également pour objet l'utilisation des acides apyrimidiques modifiés selon l'invention. Selon un mode d'utilisation, les acides nucléiques modifiés selon l'invention sont utilisés dans des réactions d'hybridation avec des acides nucléiques ou des séquences d'acides nucléiques complémentaires.

On a en effet constaté avec surprise que les modifications décrites plus haut conservent aux acides apyrimidiques modifiés toute leur faculté de s'apparier à des acides nucléiques complémentaires, et ceci même lorsqu'ils sont fragmentés. Il est intéressant de noter que dans ce cas l'hybridation est même facilitée.

Les acides nucléiques modifiés selon l'invention peuvent donc être utilisés pour détecter, localiser, identifier, voire même doser, des acides nucléiques ou des fragments d'acides nucléiques, notamment des gènes. Ils peuvent en particulier être utilisés comme sondes «marquables» pour le diagnostic d'anomalies génétiques ou la détection d'agents pathogènes.

Les combinaisons résultant de la fixation, selon l'invention, d'un acide apyrimidique modifié sur un support solide constitué par exemple de nylon, cellulose, agarose ou latex peuvent être utilisées pour retenir sélectivement des entités présentant une affinité pour l'acide nucléique ayant subi la modification. Ils peuvent donc servir à purifier les enzymes utilisant des acides nucléiques comme substrat telles que notamment les nucléases, les polymérases et les gyrases. Ils peuvent également être utilisés pour la constitution

de filtres rentenant spécifiquement des gènes ou des ARN messagers. Dans ce dernier cas, le filtre comporte avantageusement des poly-uridylates ou des poly-thymidylates modifiés selon la technique décrite et liés à des filtres de nylon ou de cellulose.

Pour la détection, la localisation, l'identification et le dosage d'acides nucléiques ou de leurs fragments, complémentaires des acides nucléiques modifiés selon l'invention, on fera en sorte que le groupe de couplage soit détecté, par exemple par une molécule affine ou un anticorps spécifique de ce groupe. Dans ce dernier cas, l'anticorps est avantageusement marqué ou lui-même susceptible d'être reconnu par un autre anticorps marqué par une enzyme, détectable par son action sur un substrat, ou par une molécule immunofluorescente.

La réaction immunoenzymatique choisie parmi les différents types bien connus de l'homme du métier, peut être plus ou moins complexe. Elle peut en particulier faire intervenir plusieurs anticorps et/ou plusieurs enzymes et substrats d'enzymes.

Selon un mode simple de réalisation, cette réaction ne fait intervenir qu'un anticorps, spécifique de l'haptène ou de l'antigène servant de substituant, et qu'une enzyme, liée à l'anticorps. A titre d'exemple, on indique que le groupe de couplage peut être de la biotine qui, selon un mode de réalisation simple, réagit de façon sélective avec de l'avidine marquée par une enzyme, laquelle réagissant avec un substrat donne directement ou non une réaction détectable ou mesurable. Cette réaction peut être avantageusement, entre autres, une réaction colorée, une réaction conduisant à l'émision de lumière («bioluminescence») ou une réaction conduisant à la formation d'un composé facilement identifiable ou dosable.

Avantageusement, l'enzyme est constituée par la 3-galactosidase qui donne une réaction colorée avec l'orthonitrophénol galactoside (ONPG).

Selon un mode de réalisation avantageux, le groupe de couplage est la biotine qui réagit avec un mélange avidine/polymères de phosphatases alcalines biotinylées préparés selon la méthode de J.J. Leary et Coll. décrite dans Proc. Nat. Acad. Sci. US 80, (1983), p. 4045-4049, pour donner un complexe qui peut être mis en évidence au moyen du chromogène commercial de la société Sigma, appelé FAST BLUE RR Salt (Sigma F 0500), selon les indications du fabricant (Bulletin Technique n° 85).

Selon un autre mode de réalisation dans lequel le groupe de couplage est la biotine, celle-ci réagit sélectivement avec des anticorps anti-biotine marqués par une enzyme dont le rôle est le même que précédemment.

Selon encore un autre mode de réalisation, le groupe de couplage est le groupement dinitrophényle (DNP) qui peut être introduit selon une méthode habituelle, par exemple par réaction de l'acide nucléique modifié par l'hydrazine avec le 1-fluoro-2,4-dinitrobenzène en présence de bicarbonate de sodium. Le groupement DNP est reconnu par des anticorps anti-DNP marqués, par exemple par une enzyme.

En tout état de cause, lorsqu'on se propose de réaliser une sonde ou un filtre comprenant un acide nucléique modifié selon l'invention, il convient de s'assurer que le groupe de couplage porté par cette sonde ou le support du filtre n'empêche pas l'hybridation ultérieure de la sonde ou, selon le cas, une réaction ultérieure de l'acide nucléique modifié avec la molécule ayant pour lui une affinité sélective.

L'utilisation des acides nucléiques modifiés selon l'invention permet une détermination rapide de la présence ou non dans un échantillon biologique de l'acide nucléique ou du gène complémentaire de la sonde utilisée, et ce même en présence d'une quantité considérable d'autres acides nucléiques. Il est même possible, après une purification suffisante de l'hybride, d'obtenir une indication quant à la concentration de l'échantillon biologique étudié en l'acide nucléique recherché ou quant au taux de répartition du gène recherché dans un ADN purifié, par dosage de l'activité, notamment enzymatique, constatée.

Partant d'un échantillon d'acide nucléique à étudier, on peut d'abord réaliser l'hybridation avec la sonde modifiée, la séparation ou la dégradation de l'éventuel excès de sonde non hybridée, la réaction de couplage entre les groupes de couplage portés par la sonde hybridée, d'une part, et le marqueur, par exemple une enzyme, dont l'éventuelle présence sera détectée, de façon en soi connue, par son action sur un substrat approprié, d'autre part.

L'invention concerne encore des trousses ou «kits» contenant l'ensemble des réactifs essentiels à la mise en œuvre de l'utilisation des acides nucléiques modifiés selon l'invention. Ces trousses peuvent en particulier contenir un échantillonnage de sondes correspondant par exemple aux ADN des virus ou bactéries, des virus ou bactéries pathogènes classiquement recherchés, voire même des sondes relatives à des gènes particuliers que devraient normalement contenir les échantillons biologiques, notamment sanguins, à tester.

A ce titre, l'invention concerne donc un nécessaire ou «kit», caractérisé en ce qu'il comporte:

- au moins une sonde spécifique, formée d'un acide nucléique modifié selon l'invention, et portant des groupes de couplage déterminés, caractéristique d'une séquence d'acide nucléique ou d'un acide nucléique à rechercher,
- une entité marquée notamment par une enzyme ou une molécule fluorescente ou luminescente ou un ensemble d'entités dont au moins une est marquée, capable de former un complexe avec le groupe de couplage de l'acide nucléique modifié,
- éventuellement le ou les substrats spécifiques de la ou les enzymes éventuelles,
- les réactifs nécessaires à la lyse du milieu cellulaire à étudier, notamment sanguin, et à l'extraction des acides nucléiques des cellules de ce milieu, ainsi qu'à la dénaturation des acides nucléiques devant être mis en jeu.

Bien entendu, l'invention peut être mise en œuvre dans d'autres domaines d'application, notamment pour le marquage de certains fragments d'ADN dans les expériences génétiques bien connues visant à établir le génotype de l'ADN en question. En particulier, l'invention peut être appliquée à la détermination de l'incorporation ou non d'un fragment d'ADN particulier dans des expériences de tri génétique comportant par exemple des opérations de transformation de l'ADN d'une cellule infectée avec un ADN étranger contenant le fragment d'ADN en question ou au contraire des opérations de transduction comportant l'incorporation d'un fragment d'ADN en question, normalement contenu dans l'ADN de la cellule, dans l'ADN du virus utilisé pour l'infection de la cellule, etc., dans la mesure où, bien entendu, on dispose d'une sonde constituée par le fragment d'ARN ou d'ADN complémentaire du fragment d'acide nucléique recherché.

La description qui suit d'un essai réalisé au laboratoire a uniquement pour but d'illustrer la préparation et l'utilisation d'un acide nucléique modifié selon l'invention.

Les essais ont été effectués avec de l'ADN de phage λ. L'ADN est dissous dans de l'eau pour obtenir une concentration de 0,5 μg/μl. 5 aliquotes de 20 μl sont utilisées: 1 aliquote est gardée comme témoin et les 4 autres sont traitées avec les quantités croissantes d'hydrazine anhydre ci-après, d'abord pendant 5 minutes à 0°C, puis 35 minutes à 20°C.

| N° de l'aliquote | hydrazine (μl) |
|---|---|
| 1 | - (témoin) |
| 2 | 5 |
| 3 | 10 |
| 4 | 20 |
| 5 | 40 |

A la fin du traitement, l'ADN modifiée par l'hydrazine est séparée selon l'un ou l'autre des modes opératoires 1 ou 2 suivants:

1. L'ADN est précipité à l'éthanol froid en ajoutant 750 μl d'éthanol et 200 μl d'acétate de sodium 0,3 m, pH7 à chaque aliquote et en maintenant 20 minutes à −80°C. Chaque aliquote est ensuite centrifugée à 10 000 g pendant 10 minutes. Le surnageant est séparé, et le culot est rincé à l'éthanol puis séché sous vide.
2. Chaque aliquote est soumise à une extraction à l'éther. L'ADN reste en solution aqueuse et l'hydrazine passe dans l'éther, qui est décanté.

La séparation de l'ADN étant effectuée selon le mode opératoire 1 ou 2, chaque culot est redissous dans 70 μl de tampon citrate de sodium (10 mM, pH7). Dans le tube témoin, le volume et la molarité en tampon sont ajustés aux mêmes valeurs.

L'urée produite dans la réaction est éliminée par passage sur tamis moléculaire (par exemple le polysaccharide réticulé tridimensionnel commercialisé par la société Pharmacia sous la dénomination Sephadex G50).

On ajoute à chaque tube 2 μl d'une solution de biotine activée (sulfo-N-hydroxysuccinimide-biotine commercialisée par la société Pierce) à 20 μg/μl dans du diméthylformamide. On laisse réagir à 37°C pendant 16 heures, puis 10 arrête la réaction par addition de 5 μl de glycine à 20% dans l'eau. On purifie l'acide biotinylé ainsi obtenu par passage sur tamis moléculaire, par exemple du Sephadex G50.

La présence de biotine couplée à l'ADN est vérifiée en déposant des gouttes de 1 μl de plusieurs dilutions du contenu de chaque tube sur des filtres de nitrocellulose (dépôts de 1 ng à 100 pg d'ADN).

Les filtres sont séchés, saturés en protéines pour éviter des absorptions parasites ultérieures d'avidine, puis trempés dans une solution d'avidine (AvDH, Vector Laboratories) et de polymères de phosphatases alcalines biotinylées (préparés selon la méthode de J. J. Leary et Coll. décrite dans Proc. Nat. Acad. Sci. USA 80. (1983). p. 4045-4049).

Après rinçage, les filtres sont traités avec le chromogène «Fast Blue RR Salt» (Sigma F 0500) selon les indications du fabricant (Bulletin technique n° 85). Seuls les dépôts faits avec de l'ADN traité à l'hydrazine se colorent et l'intensité de la coloration est proportionnelle à la quantité d'hydrazine utilisée, c'est-à-dire à la quantité de groupements $-NH_2$ créés, donc au degré de biotinylation.

L'aptitude de l'ADN biotinylé à se réapparier est vérifiée en utilisant comme sonde de l'ADN moyennement et fortement biotinylé (aliquotes n° 2 et 4). et comme cible de l'ADN dénaturé de phage λ déposé sur des filtres de nitrocellulose. De l'ADN dénaturé de sperme de hareng qui n'a pas de séquence hybridable avec l'ADN traité, est déposé sur les mêmes filtres comme témoin négatif.

4 dépôts de 320, 160, 80 et 40 ng respectivement sont faits avec chaque ADN. Un filtre sans sonde est traité dans les mêmes conditions, comme témoin. Après hybridation à 65°C pendant 17 heures, exposition au mélange avidine/polymères de phosphatases alcalines biotinylées et dépôt du chromogène, seuls les dépôts faits avec de l'ADN de phage λ et hybridés avec de l'ADN de phage λ biotinylé selon l'invention se colorent, l'intensité étant plus forte avec la sonde fortement biotinylée.

L'évaluation du seuil de détection a été faite de la façon suivante: les filtres de nitrocellulose ont été préparés avec 8 dépôts d'ADNs dénaturés de phage λ, de 250 ng à 3 pg par dilutions successives au 1/5, et un dépôt de contrôle de 250 ng d'ADN de sperme de hareng dénaturé. Après hybridation pendant 17 heures à 65°C, comme indiqué plus haut, seuls les dépôts faits avec de l'ADN de phage λ sont visibles, jusqu'au dépôt de 16 picogrammes inclus.

Comme il va de soi, l'invention concerne également les variantes possibles des modes de réalisation préférés, qui ont été indiqués ci-dessus, en particulier entreraient dans le champ de revendications qui suivent les acides nucléiques modifiés dans lesquels les groupes thymine ou uracile et les groupes cytosine seraient intacts et/ou ce serait les groupes adénosine ou guanosine qui seraient remplacés par des fragments de chaînes porteurs des groupes réactifs, l'ossature générale des acides nucléiques polymères étant par ailleurs conservée comme le serait également leur capacité d'hybridation avec des séquences nucléiques complémentaires.

Il va encore de soi qu'entrerait dans le champ des revendications tout acide apyrimidique modifié dans lequel les deux hydrogènes initialement portés par les groupes aminés des acides apyrimidiques initiaux seraient substitués.

Sont de même inclus dans le champ des revendications à titre d'équivalents tous les acides apyrimidiques modifiés dans lesquels les groupes

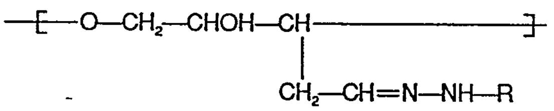

porteraient des substituants supplémentaires ou dans lesquels certains des groupes internes seraient remplacés par d'autres, pour autant que de telles modifications n'entraîneraient pas une diminution sensible de la capacité de l'acide apyrimidique ainsi modifié de s'hybrider avec une séquence nucléotidique complémentaire.

Sont encore tout particulièrement inclus dans le champ des revendications les acides nucléiques selon l'invention répondant à la formule III dans lesquels m ou n est O, en raison de la coupure, par exemple par la pipéridine, d'un acide nucléique au niveau d'une base pyrimidique modifiée par l'hydrazine.

**Revendications**

1. Application des acides apyrimidiques à la constitution de sondes servant à la détection et le cas échéant, à la séparation des séquences nucléotidiques d'acides nucléiques complémentaires contenus dans un milieu, par hybridation desdits acides apyrimidiques avec lesdites séquences nucléotidiques.

2. Application selon la revendication 1, caractérisée en ce que ces acides apyrimidiques sont modifiés par des groupes de couplage conjugués de façon covalente avec des fonctions réactives portées par ces acides apyrimidiques, ces groupes de couplage étant à leur tour capables d'intervenir dans une réaction chimique en vue, soit de la fixation sur une autre molécule ou entité chimique ou immunologique ou sur un support comportant une fonction réactive appropriée, soit de la détection d'un acidenucléique complémentaire.

3. Acide nucléique modifié constitué par un acide apyrimidique modifié dérivé d'une chaîne d'acide désoxyribonucléique (ou ribonucléique) et caractérisée par le remplacement d'une partie au moins des groupes désoxycytidine-phosphate (ou cytidine-phosphate) et, le cas échéant, d'au moins une partie des groupes thymidine-phosphate (ou uridine-phosphate) par des groupes de formule générale II:

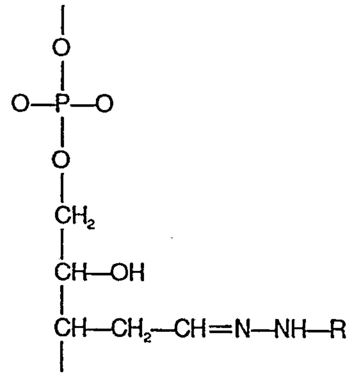

(II)

reliés dans la chaîne à des groupes semblables ou à des nucléotides intacts, R repésentant un groupe de couplage.

4. Acide nucléique modifié selon la revendication 3, caractérisé par la formule générale III:

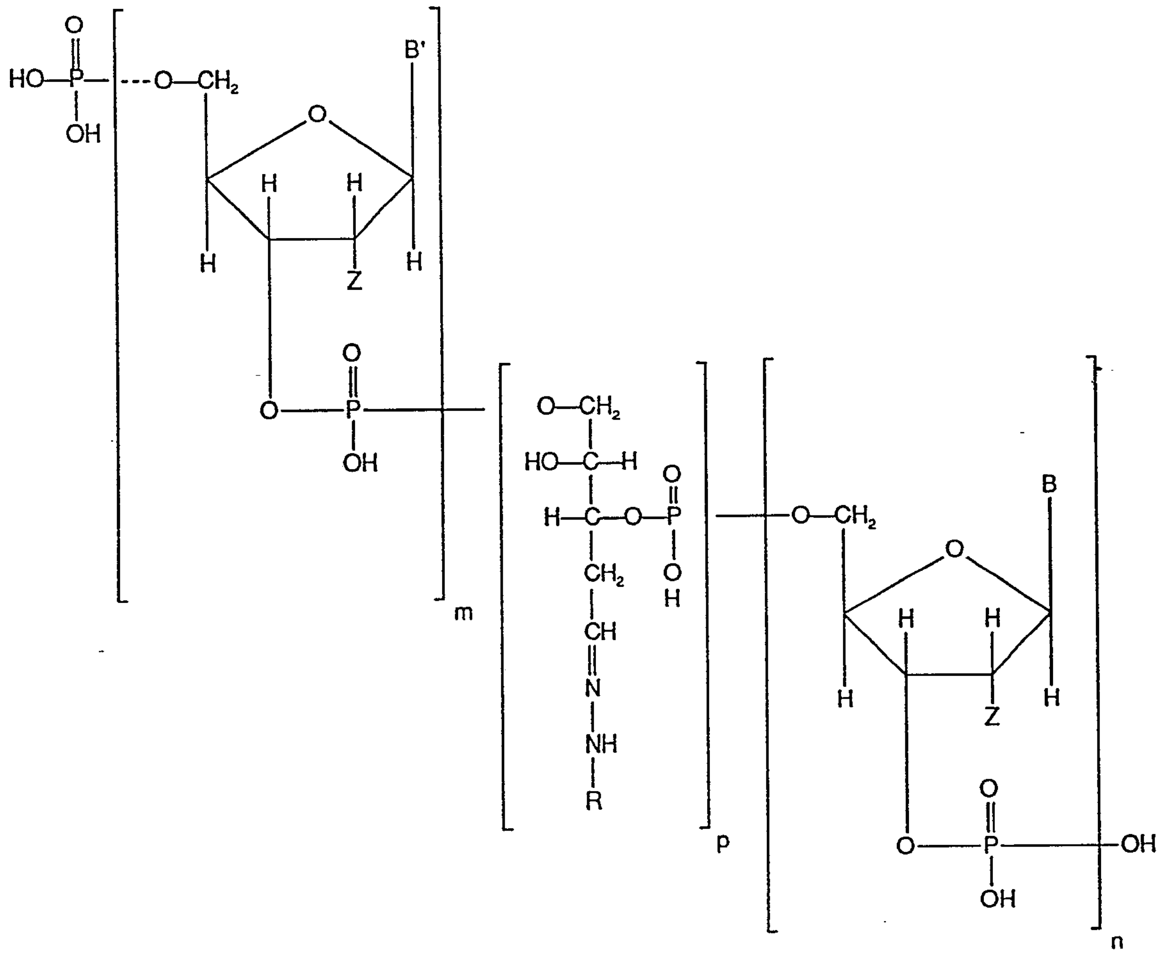

(III)

dans laquelle:

- chacune des lettres B, B′ ... représente un groupe purine, 7-déazapurine ou pyrimidine, chacun de ces groupes B, B′ ... étant lié de façon covalente au groupe sucre correspondant en la position $C^1$ de celui-ci, soit au niveau de sa propre position $N^9$, pour les groupes purine ou 7-déazapurine, soit au niveau de sa propre position $N^1$ pour les groupes pyrimidine,
- R représente un groupe de couplage reconnaissable par une molécule affine, une polypeptide ou un anticorps, ou encore un groupe permettant le couplage covalent avec un autre groupe fonctionnel porté soit par une autre molécule, soit par un support,
- z représente -H ou -OH et
- m et n représentent chacun 0 ou des nombres entiers et la somme m+n est avantageusement comprise entre 100 et 10 000, de préférence comprise entre 300 et 2000,
- p est un nombre entier au moins égal à 1 et le rapport

$$\frac{p}{m+n+p}$$

est avantageusement compris entre 0,01 et 0,05.

5. Acide nucléique modifié selon la revendication 3 ou la revendication 4, qui est constitué par un ADN et qui est caractérisé en ce que la totalité des groupes thymidines est intacte.

6. Acide nucléique modifié selon l'une quelconque des revendications 3 à 5, caractérisé en ce que le groupe de couplage correspond à un groupe auquel correspond une molécule présentant une affinité spécifique à l'égard de ce groupe.

7. Acide nucléique modifié selon la revendication 6, caractérisé en ce que le groupe de couplage présente les propriétés d'un haptène à l'égard d'un anticorps présentant une affinité sélective pour ce groupe de couplage.

8. Application de l'acide nucléique modifié selon l'une des revendications 3 à 7, à la constitution de sondes non radio-actives pour la détection d'ADNs ou séquences spécifiques d'ADNs éventuellement contenues dans une composition d'acides nucléiques, la sonde hybridée étant détectée par couplage, direct ou indirect, de son groupe de couplage avec une enzyme détectable par son action sur un substrat spécifique ou avec une molécule luminescente ou fluorescente.

9. Application des acides nucléiques modifiés selon l'une quelconque des revendications 3 à 7 à la fixation sur des supports en vue de la constitution de filtres ou matériaux de chromatographie d'affinité ou analogue, permettant la rétention sélective par hybridation de séquences d'acides nucléiques complémentaires contenus dans un mélange d'acides nucléiques ou de certaines enzymes.

10. Nécessaire ou «kit» pour la mise en œuvre de l'application selon l'une des revendications 1, 2, 8 ou 9, caractérisé en ce qu'il comporte:
- au moins une sonde spécifique, formée d'un acide nucléique apyrimidique, et portant des groupes de couplage déterminés, caractéristique d'une séquence d'acide nucléique ou d'un acide nucléique à rechercher,
- une entité marquée notamment par une enzyme ou une molécule fluorescente ou luminescente ou un ensemble d'entités dont au moins une est marquée, capable de former un complexe avec le groupe de couplage de l'acide nucléique modifié,
- éventuellement le ou les substrats spécifiques de la ou les enzymes éventuelles,
- les réactifs nécessaires à la lyse du milieu cellulaire à étudier, notamment sanguin, et à l'extraction des acides nucléiques des cellules de ce milieu, ainsi qu'à la dénaturation des acides nucléiques devant être mis en jeu.

## Patentansprüche

1. Verwendung von apyrimidinischen Säuren zum Aufbau von Sonden, die zum Nachweis und ggf. zur Trennung der nukleotidischen Sequenzen der komplementären Nukleinsäuren dienen, die in einem Medium enthalten sind, durch Hybridbildung dieser apyrimidinischen Säuren mit den vorgenannten nukleotidischen Sequenzen.

2. Verwendung nach Anspruch 1, dadurch gekennzeichnet, dass diese apyrimidinischen Säuren durch kopplungsfähige Gruppen modifiziert sind, die kovalent mit den reaktionsfähigen Funktionen verbunden sind, die durch diese apyrimidinischen Säuren zur Verfügung gestellt werden, wobei die kopplungsfähigen Gruppen ihrerseits in der Lage sind, in eine chemische Reaktion einzugreifen, um sich entweder auf ein anderes Molekül oder eine andere immunologische oder chemische Einheit oder eine Trägersubstanz zu fixieren, die die zweckmässige, reaktionsfähige Funktion beinhaltet, oder um eine komplementäre Nukleinsäure nachzuweisen.

3. Modifizierte Nukleinsäure, bestehend aus einer modifizierten apyrimidinischen Säure, die von einer Kette einer Desoxiribonukleinsäure oder einer Ribonukleinsäure abgeleitet ist, und gekennzeichnet ist durch den Ersatz wenigstens eines Teiles der Desoxicytidinphosphat- oder Cytidinphosphat-Gruppen und ggf. wenigstens eines Teiles der Thymidinphosphat- oder Uridinphosphat-Gruppen durch Gruppen der allgemeinen Formel II:

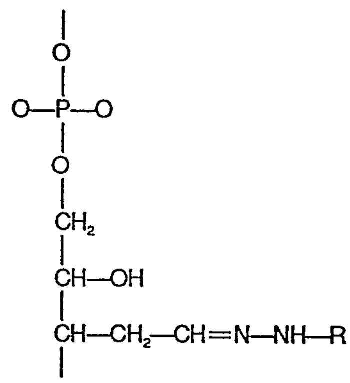

(II)

die in der Kette mit ähnlichen Gruppen oder intakten Nukleotiden verbunden sind, wobei R eine kupplungsfähige Gruppe darstellt.

4. Modifizierte Nukleinsäure nach Anspruch 3, gekennzeichnet durch die allgemeine Formel III:

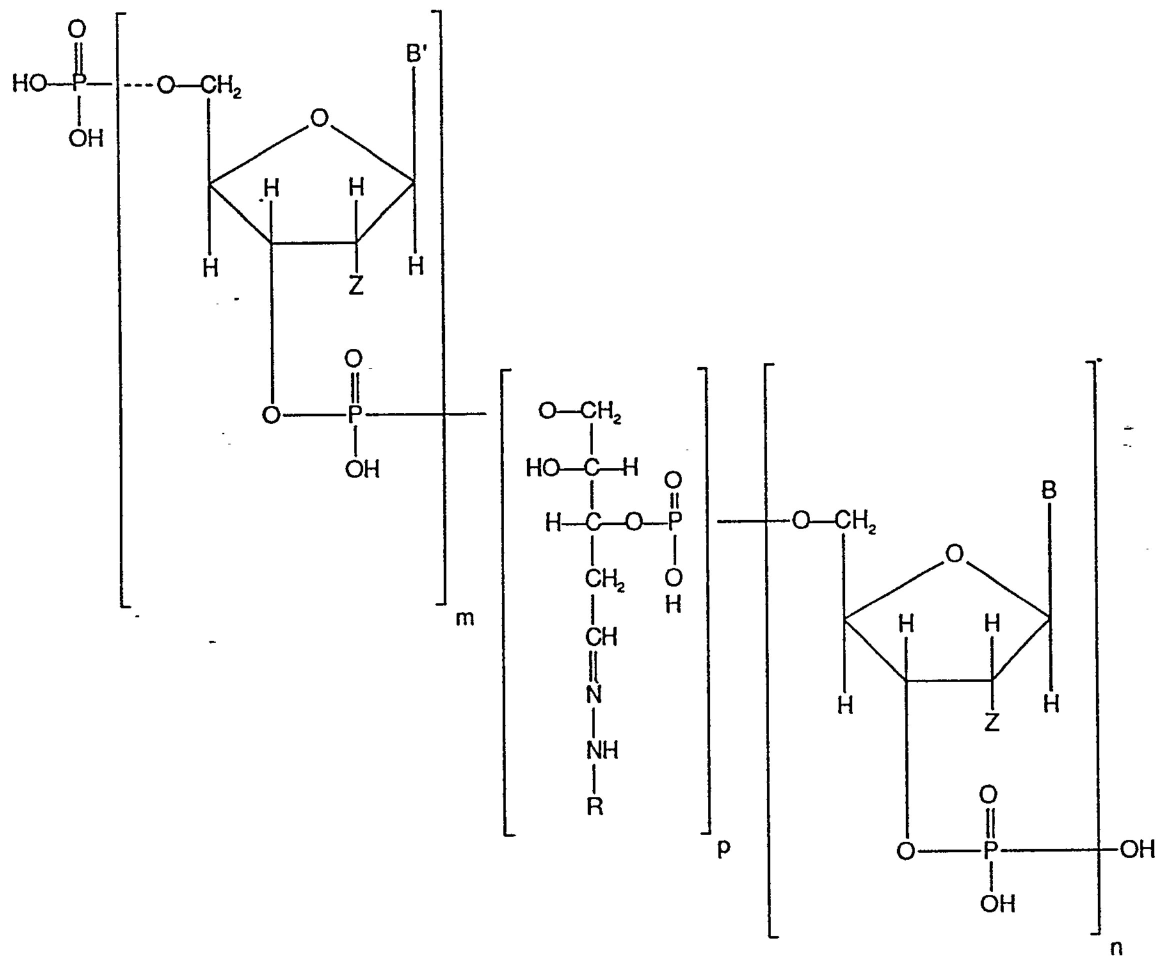

(III)

in welcher:

- jeder der Buchstaben B, B′ ... eine Purin-, eine 7-deazapurin- oder eine Pyrimidin-Gruppe darstellt, wobei jede dieser Gruppen B, B′ ... kovalent an die ihrer $C^1$-Stellung entsprechende Zukker-Gruppe gebunden ist, sei es auf dem Niveau ihrer eigenen $N^9$-Stellung, was die Purin- oder 7-deazapurin-Gruppen betrifft, sei es auf dem Niveau ihrer eigenen $N^1$-Stellung, was die Pyrimidin-Gruppen angeht,
- R eine an einem affinen Molekül erkennbare kopplungsfähige Gruppe, ein Polypeptid oder einen Antikörper, oder noch eine andere Gruppe darstellt, die eine kovalente Kopplung mit einer anderen funktionellen Gruppe gestattet, die entweder durch ein anderes Molekül oder einen anderen Träger zur Verfügung gestellt wird,
- Z eine H- oder eine OH-Gruppe darstellt und
- m und n jeweils 0 oder ganze Zahlen darstellen und die Summe m+n vorteilhafterweise zwischen 100 und 10 000 liegt, vorzugsweise zwischen 300 und 2000,
- p eine ganze Zahl mindestens gleich 1 ist und das Verhältnis

$$\frac{p}{m+n+p}$$

vorteilhafterweise zwischen 0,01 und 0,05 liegt.

5. Modifizierte Nukleinsäure nach Anspruch 3 oder Anspruch 4, die mittels einer DNS aufgebaut ist, dadurch gekennzeichnet, dass die Gesamtheit der Thymidin-Gruppen intakt ist.

6. Modifizierte Nukleinsäure nach einem der Ansprüche 3 bis 5, dadurch gekennzeichnet, dass die kopplungsfähige Gruppe einer Gruppe entspricht, die ihrerseits einem Molekül entspricht, das eine spezifische Affinität in Hinblick auf diese Gruppe aufweist.

7. Modifizierte Nukleinsäure nach Anspruch 6, dadurch gekennzeichnet, dass die kopplungsfähige Gruppe die Eigenschaften eines Haptens in bezug auf einen Antikörper aufweist, der eine selektive Affinität für die kopplungsfähige Gruppe aufweist.

8. Verwendung der modifizierten Nukleinsäure nach einem der Ansprüche 3-7 zum Aufbau für nichtradioaktive Sonden zum Nachweis von DNS oder spezifischen Sequenzen der DNS, die ggf. in einer Zusammensetzung aus Nukleinsäuren enthalten sind, wobei die hybridisierte Sonde durch direkte oder indirekte Kopplung ihrer kopplungsfähigen Gruppe mit einem durch seine Wirkung auf ein spezifisches Substrat oder ein luminiszierendes oder fluoreszierenden Molekül feststellbaren Enzym nachgewiesen wird.

9. Verwendung von modifizierten Nukleinsäuren nach einem der Ansprüche 3-7 zur Festlegung auf Träger zum Aufbau von Filtern oder affinen oder analogen chromatographischen Materialien, wobei durch Hybridbildung die Sequenzen der komplementären Nukleinsäuren, die in einer Mischung der Nukleinsäuren oder einer Mischung bestimmter Enzyme enthalten sind, selektiv zurückgehalten werden.

10. Gerät oder «Kit» für die Durchführung der Verwendung nach einem der Ansprüche 1, 2, 8 oder 9, dadurch gekennzeichnet, dass es enthält:
- wenigstens eine spezifische Sonde, gebildet aus einer apyrimidinischen Nukleinsäure, und festgelegten kopplungsfähigen Gruppen tragend, charakteristisch in einer Sequenz einer Nukleinsäure oder in einer noch zu suchenden Nukleinsäure,
- eine markierte Einheit, insbesondere durch ein Enzym oder ein fluoreszierendes oder luminiszierendes Molekül oder eine Anzahl von Einheiten, wobei mindestens eine Einheit markiert ist, fähig einen Komplex mit der kopplungsfähigen Gruppe der modifizierten Nukleinsäure zu bilden,
- gegebenenfalls das oder die spezifischen Substrate des oder der möglichen Enzyme,
- die zur Lysis des zu untersuchenden cellulären Mediums, insbesondere Blut, und zur Extraktion der Nukleinsäuren aus den Zellen des Mediums notwendigen Reagenzien ebenso wie die zur Zersetzung der Nukleinsäuren notwendigen Reagenzien, bevor sie zur Reaktion gebracht werden.

## Claims

1. The application of apyrimidinic acids to the constitution of probes used for the detection and as the case may be, for the separation of nucleotide sequences of complementary nucleic acids contained in a medium, by hybridizing said apyrimidinic acids with said nucleotide sequences.

2. The application according to claim 1, characterized in that these apyrimidinic acids are modified by coupling groups covalently conjugated with reactive functions carried by these apyrimidinic acids, these coupling groups being in turn capable of participating in a chemical reaction in order to, either fixing onto another molecule or chemical or immunological entity or onto a carrier comprising a suitable reactive function, or detecting a complementary nucleic acid.

3. A modified nucleic acid consisting of a modified apyrimidinic acid derived from a deoxyribonucleic (or ribonucleic) acid chain and characterized by the replacement of at least on part of the deoxycytidine-phosphate (or cytidine-phosphate) groups, and, as the case may be, of at least a part of the thymidine-phosphate (or uridine-phosphate) groups with groups of the general formula II:

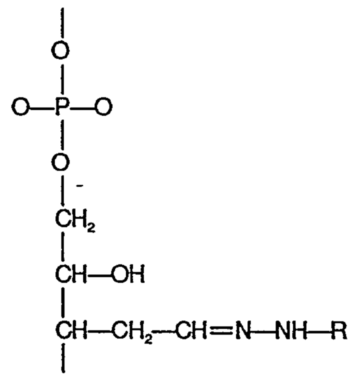

(II)

linked in the chain to similar groups or to intact nucleotides, R representing a coupling group.

4. A modified nucleic acid according to claim 3, characterized by the general formula III:

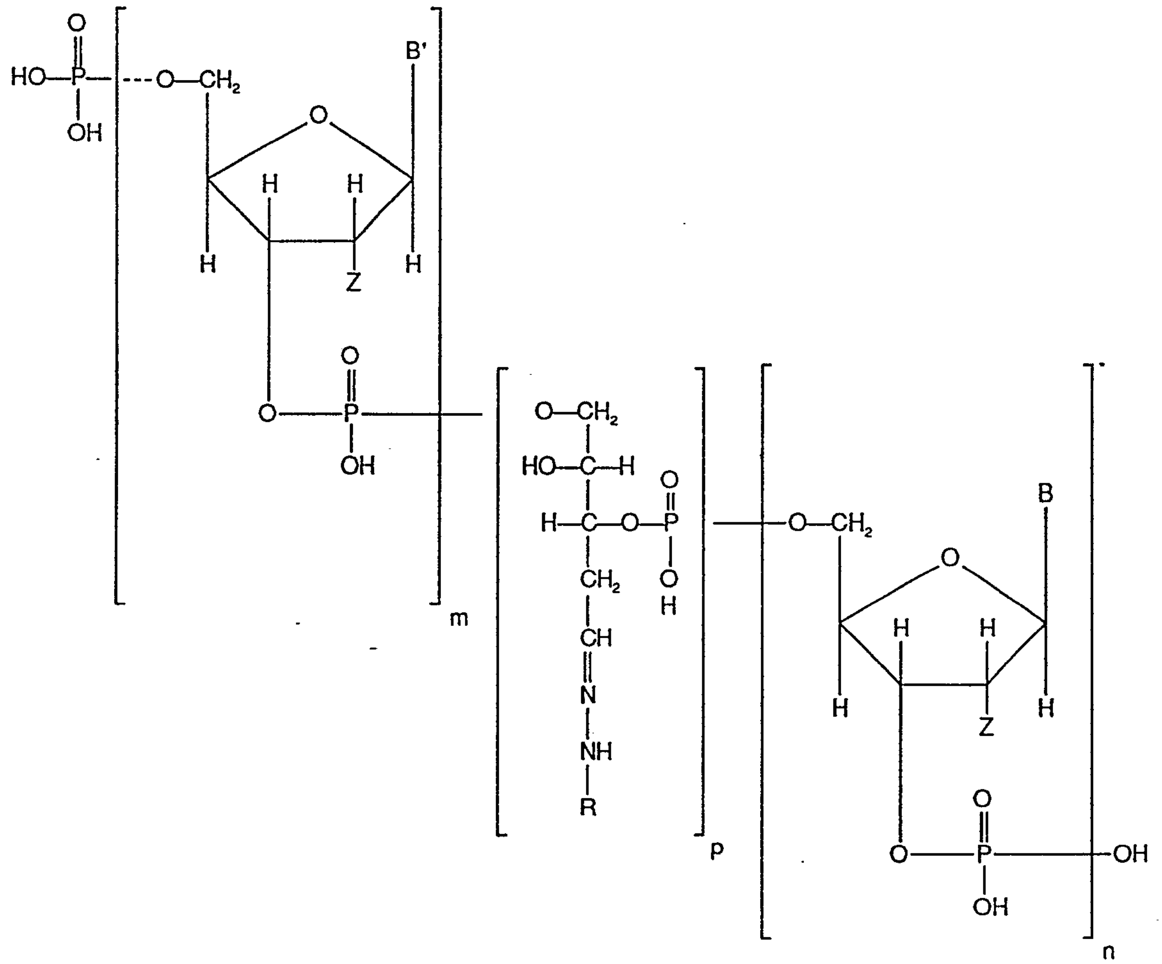

(III)

wherein:

- each of the letters B, B' ... represents a purine, 7-deazapurine or pyrimidine group, each of these groups B, B' ... being covalently linked to the corresponding sugar group in $C^1$ position thereof, either at the level of its own $N^9$ position, for the purine or 7-deazurine groups, or at the level of its own $N^1$ position for the pyrimidine groups,
- R represents a coupling group recognizable by a molecule having an affinity, a polypeptide or an antibody, or further, a group permitting the covalent coupling to another functional group carried either by another molecule, or by a carrier,
- z represents -H or -OH and
- m and n each represent 0 or integers and the sum m+n is advantageously comprised between 100 and 10 000, preferably comprised between 300 and 2000,
- p is an integer at least equal to 1 and the ratio

$$\frac{p}{m+n+p}$$

is advantageously comprised between 0.01 and 0.05.

5. A modified nucleic acid according to claim 3 or to claim 4, which is constituted by a DNA and which is characterized in that all of the thymidine groups are intact.

6. A modified nucleic acid according to any one of claims 3 to 5, characterized in that the coupling group corresponds to a group to which corresponds a molecule presenting a specific affinity for this group.

7. A modified nucleic acid according to claim 6, characterized in that the coupling group presents the properties of a hapten with respect to an antibody presenting a selective affinity for this linking group.

8. The application of the modified nucleic acid according to one of claims 3 to 7, to the constitution of non-radioactive probes for the detection of DNA's or specific sequences of DNA's, possibly contained in a composition of nucleic acids, the hybridized probe being detected by direct or indirect coupling of its coupling group with an enzyme detectable by its action on a specific substrate or with a luminiscent or fluorescent molecule.

9. The application of the modified nucleic acids according to any one of claims 3 to 7 to the fixation onto carriers for constituting filters or materials for affinity chromatography or similar, permitting the selective retention by hybridization of sequences of complementary nucleic acids contained in a mixture of nucleic acids or of certain enzymes.

10. A kit for the implementation of the application according to one of claims 1, 2, 8 or 9, characterized in that it comprises:

- at least one specific probe, consisting of apyrimidinic nucleic acid, and carrying definite coupling groups, characteristic of a nucleic acid sequence or of a nucleic acid to be investigated.

- an entity labeled, particularly by en enzyme or a fluorescent or luminescent molecule or an assembly of entities of which at least one is labeled, capable of forming a complex with the linking group of the modified nucleic acid,
- possibly, the specific substrate(s) of the possible enzyme(s),
- the reagents necessary for the lysis of the cell medium under study, in particular of blood origin, and for the extraction of the nucleic acids from the cells of this medium, as well as for the denaturation of nucleic acids which have to participate to the application.